# EUROPÄISCHE PATENTSCHRIFT

(11) **EP 1 537 830 B1**
(45) Veröffentlichungstag und Bekanntmachung des Hinweises auf die Patenterteilung: **06.09.2006**
(21) Anmeldenummer: 03028127.3
(22) Anmeldetag: 05.12.2003
(51) Int. Cl.: A61B 19/00

(54) **Verfahren und Vorrichtung zum Beobachten von Objekten mit einem Mikroskop**
Method and device for viewing an object with a microscope
Procédé et appareil pour l'observation des objets avec un microscope

(43) Veröffentlichungstag der Anmeldung: 08.06.2005
(73) Patentinhaber: Möller-Wedel GmbH, 22880 Wedel (DE)
(72) Erfinder: Schweikard, Achim, Prof. Dr., 20253 Hamburg (DE)
(74) Vertreter: Glawe, Delfs, Moll

(56) Entgegenhaltungen:
- DE-A- 4 032 207
- DE-A- 4 416 229
- DE-A- 19 640 907
- US-A- 5 359 417

## Beschreibung

Die Erfindung betrifft ein Verfahren und eine Vorrichtung zum Beobachten von Objekten aus verschiedenen Blickrichtungen mit einem Mikroskop, das eine Einrichtung zum elektronischen Speichern von Bildern und computergesteuerte Antriebseinrichtungen für mehrere Bewegungsachsen aufweist.

Mikroskope, die um mehrere Bewegungsachsen eines Stativs computergesteuert bewegt werden können, sind bekannt. Diese Mikroskope können translatorisch bewegt werden oder auch verschwenkt werden. In vielen Fällen ist es aber erwünscht, einen bestimmten Punkt des Objekts von verschiedenen Richtungen beobachten zu können. Dies spielt zwar bei zweidimensionalen Objekten keine grosse Rolle, kann aber dreidimensionalen Objekten wichtig sein, insbesondere, wenn der zu beobachtende Punkt durch näher beim Mikroskop liegende Teile des Objekts teilweise verdeckt wird. In diesem Falle ist es schwierig, die Steuerung der Bewegung des Mikroskops so durchzuführen, dass immer derselbe Punkt im Blickfeld bleibt, man ihn aber trotzdem aus unterschiedlichen Winkeln beobachten kann.

Aus der DE 4 416 229 ist ein Mikroskop der Eingangs erwähnten Art bekannt.

Die Aufgabe der Erfindung besteht in der Schaffung eines Verfahrens, mit dem eine Schwenkbewegung um den zu beobachtenden Punkt durchgeführt werden kann, ohne dass sich dieser Punkt verschiebt oder sein Bild unscharf wird.

Die erfindungsgemässe Lösung besteht darin, dass eine erste Aufnahme des Zielgebiets des Objekts erstellt und gespeichert wird, das Mikroskop um einen definierten Winkel verschwenkt wird, eine zweite Aufnahme des Zielgebiets erstellt und gespeichert wird, in beiden Aufnahmen ein und derselbe Punkt des Objekts markiert wird, durch Triangulation die Koordinaten des Punktes berechnet werden, und die Steuerung so eingerichtet wird, dass eine Schwenkbewegung um diesen Punkt durchführbar ist.

Man muss sich also zwei Winkel bzw. Blickrichtungen aussuchen, in denen ein und derselbe Punkt beobachtet werden kann. Anschliessend wird dann die Schwenkbewegung um diesen Punkt aufgrund der beiden Aufnahmen eingerichtet. Es ist dann möglich, diese Schwenkbewegung auszuführen, um Blickrichtungen bzw. Winkeleinstellungen zu finden, die den Punkt und seine Umgebung möglicherweise noch besser zeigen, als dies bei den ursprünglichen Aufnahmen der Fall war. Insbesondere bei Operationen z. B. des Gehirns können so die optimalen Beobachtungsbedingungen geschaffen werden.

Vorteilhafterweise ist die Steuerung so eingerichtet, dass nicht nur Schwenkbewegungen, sondern auch Translationsbewegungen des Mikroskops durchführbar sind. Auf diese Weise kann man andere Teile des Objekts in das Blickfeld rücken und ggf. eine Schwenkbewegung um diese Punkte einrichten.

Vorteilhafterweise wird eine Erfassung und Berechnung für mehrere Punkte des Objekts durchgeführt.

Dabei wird zweckmässigerweise ein Mikroskop mit Einrichtungen zum Einspiegeln von Daten und Bildern verwendet. Die Einrichtungen zum Einspiegeln von Daten können dabei Einrichtungen zum Einspiegeln von Daten der Bewegungssteuerung sein.

Zweckmässigerweise sind dabei die Einrichtungen zum Einspiegeln von auf andere Weise gewonnenen Bildern des Objekts ausgebildet. Auch dies ist für Operationen des Gehirns, aber nicht nur für solche Operationen vorteilhaft. Die auf andere Weise gewonnenen Bilder des Objekts können im Falle der Gehirnoperation präoperative Aufnahmen sein (z. B. MRT, CT, Röntgen, Ultraschall, PET oder Angiographiedaten). Auf diese Weise kann der Operateur Einzelheiten "sehen", die unter der für ihn durch das Mikroskop sichtbaren Oberfläche liegen. Zweckmässigerweise werden in den auf andere Weise gewonnenen Bildern des Objekts die Punkte markiert, die den in den gespeicherten Mikroskopaufnahmen markierten Bildpunkten entsprechen. Es können dann die Verschiebungen der durch das Mikroskop beobachteten Punkte relativ zu den Orten der Punkte in den auf andere Weise gewonnenen Bildern bestimmt werden. Diese Verschiebung ist bei Gehirnoperationen als "Brain Shift" bekannt. Diese Deformation der anatomischen Strukturen entsteht beispielsweise, wenn das Operationsfeld eröffnet wird und Knochen entfernt wird, um einen Zugang zu ermöglichen, oder auch wenn Teile eines Tumors zu Beginn einer Operation bereits entfernt wurden. Dadurch ist es häufig nur schwer möglich, intraoperativ Strukturen in der Anatomie den aus den dreidimensionalen präoperativen Bildern bekannten entsprechenden Strukturen zuzuordnen. Während die präoperativ aufgenommenen Bilder oft hoch aufgelöst sind und Strukturen gut sichtbar erscheinen lassen, ist dies während der Operation allein aufgrund der intraoperativ sichtbaren Oberfläche häufig schwierig. Wenn nun durch Interpolation eine Verschiebungsfunktion für die zwischen markierten Punkten angeordneten Punkte bestimmt wird, die vorteilhafterweise auch über die markierten Punkte hinaus extrapoliert wird, so können die auf andere Weise gewonnen Bilder unter Verwendung dieser Verschiebungsfunktion so deformiert werden, dass die markierten Punkte in den selben mit dem Mikroskop beobachteten entsprechenden Punkten zusammenfallen. In diesem Falle kann der Operateur trotz des Brain Shifts übereinandergelagert die selbe Stelle im Gehirn sehen, nämlich einmal direkt durch das Mikroskop und andererseits überlagert durch die präoperative Aufnahme.

Eine Vorrichtung zum Durchführen des Verfahrens mit einem Mikroskop, das eine Einrichtung zum elektronischen Speichern von Bildern und computergesteuerte Antriebseinrichtungen für mehrere Bewegungsachsen aufweist, zeichnet sich dadurch aus, dass es Einrichtungen zum Einspiegeln von Steuerungsdaten der Antriebseinrichtungen aufweist. Zweckmässigerweise sind die Antriebseinrichtungen drahtlos mit einer sterilisierbaren Einheit steuerbar, die einen Betätigungsknopf für die Bewegungsart und einen Betätigungsknopf für die Ausführung eines Bewegungsschritts aufweist. Diese sterilisierbare Einheit kann z. B. am chirurgischen Instrument angebracht sein, so dass der Operateur dieses nicht aus der Hand legen muss, wenn er das Mikroskop verstellen will. Die drahtlose Verbindung zwischen der am chirurgischen Instrument angeordneten sterilisierbaren Einheit kann z. B. eine Funkverbindung oder eine Ultraschallverbindung sein.

Die Computersteuerung kann dabei zum Bewirken von translatorischen und Schwenkbewegungen ausgebildet sein.

Durch die Einrichtung zum Einspiegeln von Steuerungsdaten werden die durch die Schalter gegebenen Voreinstellungen eingeblendet. In die Steuerung des Mikroskops sind Antriebe integriert, die eine ruckfreie automatische Nachführung des Mikroskops erlauben. Mittels einer derartigen Vorrichtung erfolgt die Nachführung des Mikroskops durch folgende Verfahrensschritte:
1. Durch Betätigen eines Schaltelements kann die nachfolgende Bewegungsaktion vorselektiert werden
2. Die dann selektierte Bewegungsaktion wird am Rand des Sichtfelds des Mikroskops angezeigt
3. Nachfolgendes Betätigen des anderen Schaltelements führt die jeweils angezeigte Bewegungsaktion aus
4. Die Bewegungsaktion wird in drei translatorische Richtungen vorgesehen
5. Für die Bewegung kann eine feste Schrittweite (z. B. 3 mm) vorselektiert werden
6. Durch geeignete robotische Steuerungsmethoden wird jeweils ein Überschiessen verhindert.
7. Statt der genannten drei translatorischen Richtungen kann auch eine Schwenkung um einen im Arbeitsraum selektierten Punkt erfolgen.

Mit dem Verfahren und der Vorrichtung der Erfindung ist es auch möglich, einen beliebigen Punkt des Objekts als neuen Schwenkpunkt festzulegen, wie dies weiter oben erläutert wurde, was im wesentlichen mit Hilfe von zwei Aufnahmen, Markierung desselben Punktes in beiden Aufnahmen und Triangulation der Koordinaten des Punktes geschieht.

Wenn die Vorrichtung mit Einrichtungen zum Markieren von Punkten sowohl in den elektronisch gespeicherten Bildern als auch in den auf andere Weise gewonnenen Bildern und Einrichtungen zum Bestimmen der Verschiebung versehen, so kann folgendermassen vorgegangen werden:

Werden mehrere Raum-Punkte auf die Art festgelegt, und sind einige dieser Punkte auch schon in präoperativen Aufnahmen (z. B. MRT, CT, Röntgen, Ultraschall, PET oder Angiographiedaten markiert), so kann eine Korrespondenz zwischen den Punkten hergestellt werden. Insbesondere kann eine mathematische Deformationsfunktion berechnet werden, die die präoperativen Punkte in die intraoperativen genau überführt. Dadurch wird es möglich, die Lage von verborgenen Punkten in der Anatomie einzuspiegeln, die gegenwärtig nicht sichtbar sind, aber in präoperativen Bildern zu erkennen sind. Insbesondere ergibt sich durch das beschriebene Verfahren eine Möglichkeit, den Brain Shift, also die intraoperative Verformung des Gewebes zu kompensieren, und trotzdem eine Navigation zu ermöglichen. Wesentlicher Vorteil des erfindungsgemässen Verfahrens ist die Möglichkeit, auf ein externes Lageverfolgungssystem zu verzichten.

Andere vorbekannte Arbeiten versuchen, den sogenannten Brain Shift durch Simulationsverfahren zu kompensieren. Dabei werden Schritte und deren Wirkung am Rechner simuliert, und die Wirkung anschliessend als kompensierende Deformation zurückgerechnet. Dieser Ansatz bietet jedoch nur sehr ungenaue Ergebnisse, da es hierzu erforderlich wäre, nur genau die geplanten Schnitte einzusetzen, was in der Praxis selten möglich ist.

Navigationsverfahren schlagen vor, die präoperativen Daten intraoperativ einzuspielen. Bekannte Verfahren verwenden hierzu ein Lageverfolgungssystem, beispielsweise ein kommerziell verfügbares infrarot-basiertes Navigationssystem. Ein solches System ist kostenaufwendig und allein nicht in der Lage, den Brain Shift zu kompensieren. Auch die Kombination mit den zuvor genannten Simulationsverfahren bietet hier nur sehr ungenaue Ergebnisse, da es auch hier erforderlich wäre, nur die geplanten Schnitte einzusetzen.

Dadurch, dass die Antriebseinrichtungen drahtlos mit einer sterilisierbaren Einheit steuerbar sind, werden die Probleme des Standes der Technik vermieden. Die Bewegung kann direkt durch den Chirurgen erfolgen. Es ist zwar eine Sprachsteuerung denkbar. Diese arbeitet aber unter OP-Bedingungen unzuverlässig und widerspricht ergonomischen Anforderungen aus der Anwendung. Bei anderen vorbekannten Einrichtungen besteht die Notwendigkeit, dass Operationsinstrument zwischenzeitlich abgelegen zu müssen. Die Bedienung durch Fuss- oder Mundschalter widerspricht ebenfalls ergonomischen Anforderungen.

Die wesentlichen Punkte der Erfindung können wie folgt zusammengefasst werden. Die Erfindung betrifft ein Verfahren zur Navigation mittels eines chirurgischen Mikroskops, bei dem die intraoperative Verformung des Gewebes während einer Operation ausgeglichen werden kann. Ausgangspunkt sind die bereits bekannten Mikroskop-Systeme, bei denen einzelne Bewegungsachsen im Trägerstativ mit computergesteuerten Antrieben ausgerüstet sind. Über am chirurgischen Instrument angebrachte sterilisierbare Schaltelemente werden Parameter einer durch das Mikroskop auszuführenden räumlichen Bewegung vorgegeben. Die durch die Schaltelemente getroffenen Einstellungen können durch Einspiegelung in das Sichtfenster des Mikroskops visualisiert und anschliessend aktualisiert werden. Die Bewegung des Mikroskops erfolgt in vorselektierten Schrittweiten. Dadurch kann das Mikroskop in einfacher Weise während der Operation nachgeführt werden. Die Nachführung erfordert die Bewegung auf einem kugelförmigen Volumen um einen festen Raumpunkt. Dieser Punkt muss bei Beginn der Operation geeignet festgelegt werden. Zur Festlegung dieses Punktes wird so vorgegangen, zunächst einen Punkt in der Zielanatomie durch einen elektronisch erfassbaren Zeiger, z. B. Mauszeiger zu markieren, dann das robotergesteuerte Mikroskop in einem festen, dem System bekannten Winkel zu verfahren, und den gleichen Punkt erneut zu markieren. Da die Relativlage der beiden Bilder bekannt ist, kann die genaue räumliche Lage dieses Punktes errechnet werden. In gleicher Weise werden nun mehrere solcher Punkte erfasst. Sind in einem präoperativ erfassten Datensatz nun die gleichen Punkte markiert, so kann die mathematische Deformationsfunktion errechnet werden, die die präoperative Punktmenge in die intraoperative überführt. Dadurch kann anschliessend weitere präoperativ markierte oder erfasste Information ins Sichtfenster des Mikroskops eingespiegelt werden. Insbesondere kann während der Operation die genaue aktuelle Lage von unter der Oberfläche verborgenen Strukturen visualisiert werden, ohne dass ein externes Navigationssystem (Tracking-System) erforderlich ist.

Die Erfindung wird im Folgenden beispielsweise erläutert. Es zeigen:
- Figur 1: die in das Bildfeld des Mikroskops eingespiegelten Bewegungsparameter;
- Figur 2: schematisch die Anwendung des Verfahrens der Erfindung;
- Figur 3: schematisch den Strahlungsgang eines erfindungsgemässen Mikroskops; und
- Figur 4-6: Darstellung eines Mikroskopstativs mit den erfindungsgemässen Antriebseinrichtungen.

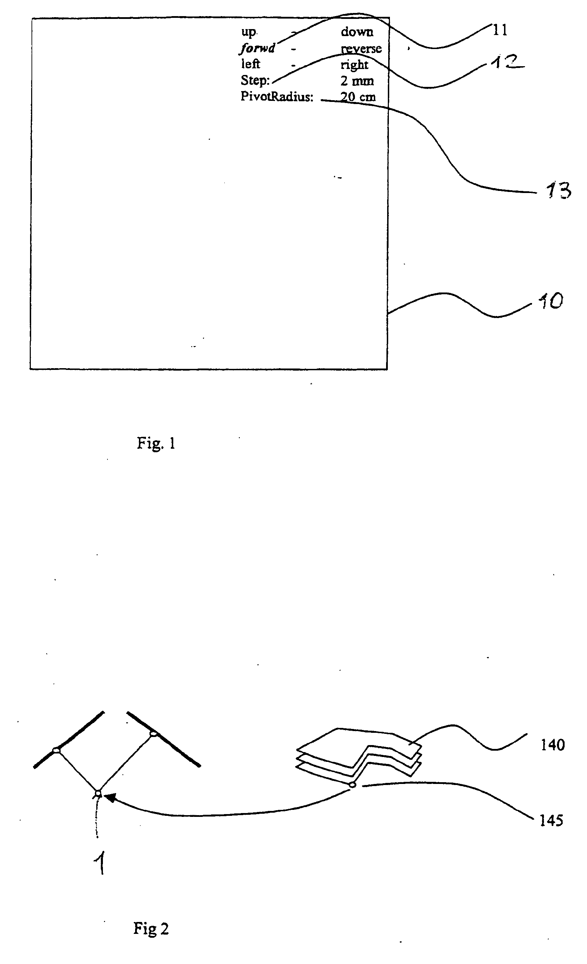

In Figur 1 ist schematisch das Bildfeld 10 eines Mikroskops gezeigt, in das die verschiedenen Bewegungsmöglichkeiten eingespiegelt sind. Die gerade gewählte Bewegungsrichtung ist dabei bei 11 hevorgehoben (heller, farblich abgesetzt oder kursiv). Die Schrittweite ist bei 12 dargestellt, während der Schwenkradius bei 13 dargestellt ist.

In Figur 2 ist links die Beobachtung eines Punktes 1 unter zwei verschiedenen Blickwinkeln angedeutet. Durch Triangulation kann dann der genaue Ort dieses Punktes 1 festgestellt werden. Der entsprechende Punkt 145 in auf andere Weise gewonnenen Bildern (in der Figur sind mehrere Schichtdarstellungen 140 gezeigt, die z. B. durch Magnetresonanztomographie gewonnen worden sind) entspricht dabei dem Punkt 1 und kann mit diesem mathematisch korreliert werden, um den Brain Shift zu kompensieren.

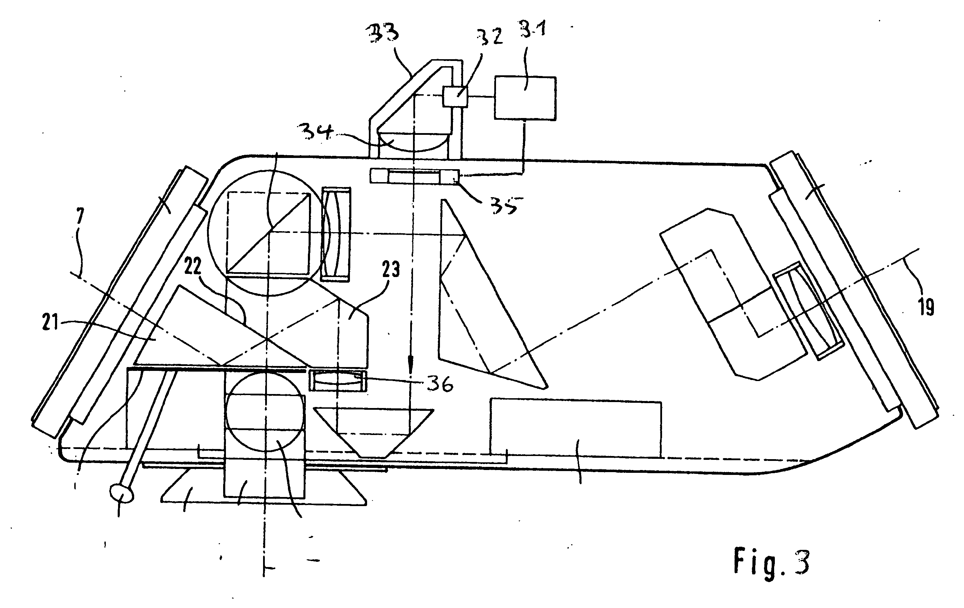

Das in Fig. 3 schematisch dargestellte Mikroskop weist eine Projektionseinheit auf, in der mit Hilfe von elektronischen Schaltungen 31 ein Bild erzeugt werden kann. Die Schaltung 31 speist dabei auch eine Lichtquelle 32 in Form z. B. einer Lampe oder einer LED, deren Licht bei 33 in eine Kollimatorlinse 34 z. B. diffus umgelenkt wird, die das Licht durch ein Durchsicht-Display 35 sendet, in der mit Hilfe der Schaltung 31 ein Bild erzeugt wird. Dieses Bild wird über eine Linse 36 und weitere in Fig. 1 gezeigte optische Elemente in ein Prisma 23 geleitet, das mit dem Prisma 21 zusammengebaut ist und die Strahlenteilerfläche 22 gemeinsam hat. Ein Teil des von der Projektionseinheit 31-35 kommenden Lichts wird dabei in den Strahlengang 7 für den Hauptbeobachter gesandt, während der andere Teil entweder zu einer Anschlusseinrichtung für den Mitbeobachtertubus in den Strahlengang 19 für den Mitbeobachter oder aber zusammen mit dem Hauptstrahlengang seitlich herausgelenkt wird.

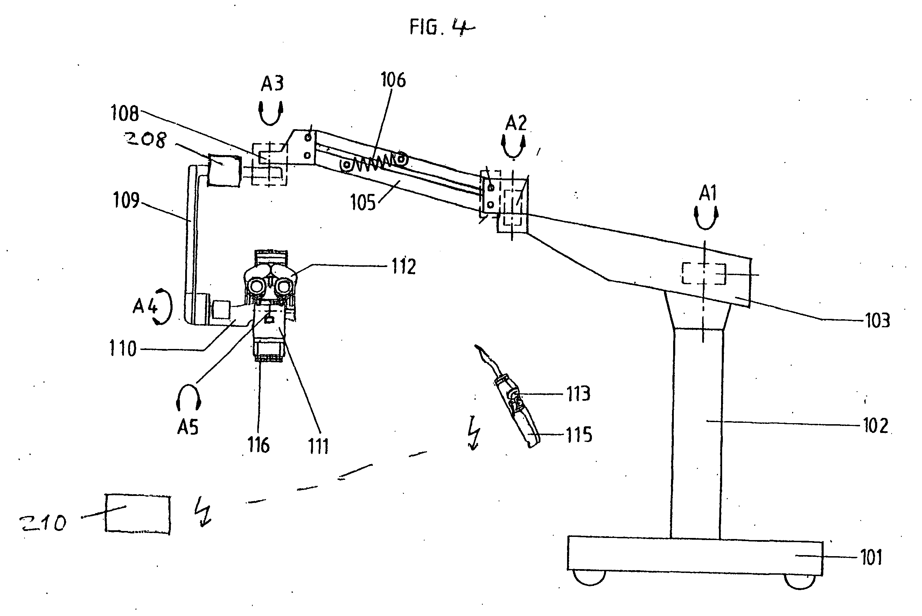

In Fig. 4 ist die Anordnung eines Operationsmikroskops an einem Fussbodenstativ dargestellt. Das Stativ steht auf einem Basisteil oder Fuss 101, der im allgemeinen mit Rollen zum Verfahren ausgestattet ist. Selbstverständlich kann der Basisteil 101 auch für Decken- oder Wandbefestigung ausgebildet sein. Auf diesem Basisteil 101 ist eine Säule 102 angebracht, um deren Achse A1 sich der aufgesetzte feste Arm 103 drehen lässt. Mit einem um die Achse A2 drehbaren Gelenk ist daran ein Parallelogrammarm 105 befestigt. Die Höhenverstellung des Mikroskopanschlusses 108 am Parallelogramm 105 ist mit einer Gasfeder oder einem Federpaket 106 gewichtsausgeglichen.

Das Mikroskop besteht aus einem Mikroskopkörper 111, dem Einblick 112 und dem Objektiv 116 und ist mit den Armen 110 und 109 drehbar um die Achse A3 am Mikroskopanschluss des Stativs befestigt. Es lässt sich mit den Drehungen um die Achsen A1, A2 und A3 sowie durch die gewichtsausgeglichene Höhenverstellung mit dem Parallelogrammarm 105 im mechanisch vorgegebenen Raum bis auf Reibungswiderstände frei von Schwerkrafteinfluss bewegen.

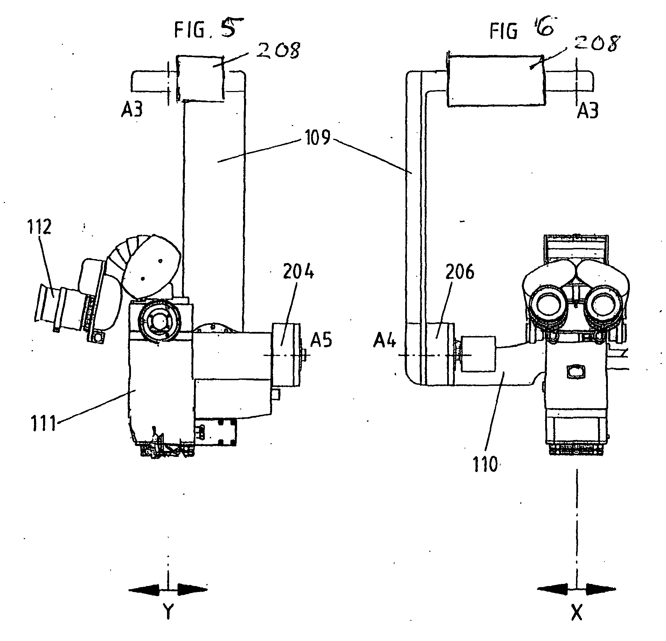

Wie dies in den Fig. 4-6 dargestellt ist, ist das dort dargestellte Mikroskop 111 mit dem Antrieb 204 um die Achse A5 drehbar. Dies entspricht einer Drehung im Sehfeld in X-Richtung. Zusammen mit dem Arm 110 kann das Mikroskop 111 vom Antrieb 206 um die Achse A4 gedreht werden. Dies entspricht einer Bewegung im Sehfeld nach oben/unten (Y-Richtung).

Zusätzlich zu den Antriebseinrichtungen 204 und 206 für die Achsen A5 und A4 ist eine Antriebseinrichtung 208 vorgesehen, mit der eine Verstellung des Mikroskoparms 109 und damit des Mikroskops 111 in drei verschiedenen zueinander senkrechten Richtungen möglich ist. Die Steuerung erfolgt dabei über eine in Fig. 4 schematisch dargestellte Steuereinheit 210, die über nicht gezeigte Kabel mit den Verstelleinrichtungen 204, 206 und 208 verbunden ist. Die Steuereinheit 210 wird dabei durch eine mit dem chirurgischen Instrument verbundene Fernsteuereinheit 115 mit Betätigungstasten 113 gesteuert.

Die Berechnungen, z. B. zum Bestimmen der Verschiebungsfunktion durch Interpolation und/oder Extrapolation und zum Deformieren der auf andere Weise gewonnenen Bilder erfolgt ebenfalls in der Steuereinheit 210. Die Markierung von Punkten kann z. B. durch Betätigung der Tasten 113 der drahtlosen Steuerung 115 erfolgen.

## Patentansprüche

1. Verfahren zum Beobachten von Objekten aus verschiedenen Blickrichtungen mit einem Mikroskop, das eine Einrichtung zum elektronischen Speichern von Bildern und computergesteuerte Antriebseinrichtungen für mehrere Bewegungsachsen aufweist, **dadurch gekennzeichnet, dass**
- eine erste Aufnahme des Zielgebiets des Objekts erstellt und gespeichert wird,
- das Mikroskop um einen definierten Winkel verschwenkt wird,
- eine zweite Aufnahme des Zielgebiets erstellt und gespeichert wird,
- in beiden Aufnahmen ein und derselbe Punkt des Objekts markiert wird,
- durch Triangulation die Koordinaten des Punkts berechnet werden, und
- die Steuerung so eingerichtet wird, dass eine Schwenkbewegung um diesen Punkt durchführbar ist.

2. Verfahren nach Anspruch 1, **dadurch gekennzeichnet, dass** die Steuerung so eingerichtet wird, dass Translationsbewegungen des Mikroskop durchführbar sind.

3. Verfahren nach Anspruch 1 oder 2, **dadurch gekennzeichnet, dass** die Erfassung und Berechnung für mehrere Punkte des Objekts durchgeführt wird.

4. Verfahren nach einem der Ansprüche 1 bis 3, **dadurch gekennzeichnet, dass** ein Mikroskop mit Einrichtungen zum Einspiegeln von Daten und Bildern verwendet wird.

5. Verfahren nach Anspruch 4, **dadurch gekennzeichnet, dass** die Einrichtungen zum Einspiegeln von Daten der Bewegungssteuerung ausgebildet sind.

6. Verfahren nach Anspruch 4 oder 5, **dadurch gekennzeichnet, dass** die Einrichtungen zum Einspiegeln von auf andere Weise gewonnenen Bildern des Objekts ausgebildet sind.

7. Verfahren nach Anspruch 6, **dadurch gekennzeichnet, dass** in den auf andere Weise gewonnenen Bildern des Objekts die Punkte markiert werden, die den in den gespeicherten Mikroskopaufnahmen markierten Bildpunkten entsprechen.

8. Verfahren nach Anspruch 7, **dadurch gekennzeichnet, dass** Verschiebungen der durch das Mikroskop beobachteten Punkte relativ zu den Orten der Punkte in den auf andere Weise gewonnenen Bildern bestimmt werden.

9. Verfahren nach Anspruch 8, **dadurch gekennzeichnet, dass** durch Interpolation eine Verschiebungsfunktion für die zwischen den markierten Punkten angeordneten Punkte bestimmt wird.

10. Verfahren nach Anspruch 9, **dadurch gekennzeichnet, dass** die Verschiebungsfunktion über die markierten Punkte hinaus extrapoliert wird.

11. Verfahren nach Anspruch 9 oder 10, **dadurch gekennzeichnet, dass** die auf andere Weise gewonnenen Bilder unter Verwendung der Verschiebungsfunktion so deformiert werden, dass die markierten Punkte in den derselben mit den mit dem Mikroskop beobachteten entsprechenden Punkten zusammenfallen.

12. Vorrichtung zum Durchführen des Verfahrens nach einem der Ansprüche 1 bis 11 mit einem Mikroskop, das eine Einrichtung zum elektronischen Speichern von Bildern und computergesteuerte Antriebseinrichtungen für mehrere Bewegungsachsen aufweist, **dadurch gekennzeichnet, dass** sie Einrichtungen (31-36) zum Einspiegeln von Steuerungsdaten (11, 12, 13) der Antriebseinrichtungen (204, 206, 208) aufweist.

13. Vorrichtung nach Anspruch 12, **dadurch gekennzeichnet, dass** die Antriebseinrichtungen (204, 206, 208) drahtlos mit einer sterilisierbaren Einheit (115) steuerbar sind, die einen Betätigungsknopf (113) für die Bewegungsart und einen Betätigungsknopf (113) für die Ausführung eines Bewegungsschrittes aufweist.

14. Vorrichtung nach Anspruch 12 oder 13, **dadurch gekennzeichnet, dass** die Computersteuerung (210) zum Bewirken von translatorischen und Schwenkbewegungen ausgebildet ist.

15. Vorrichtung nach einem der Ansprüche 12 bis 14, **dadurch gekennzeichnet, dass** sie eine Einrichtung zum Einspiegeln von auf andere Weise gewonnenen (31-36) Bildern (140) des Objekts in das Bildfeld (10) des Mikroskops aufweist.

16. Vorrichtung nach Anspruch 15, **dadurch gekennzeichnet, dass** sie Einrichtungen (113) zum Markieren von Punkten in den elektronisch gespeicherten Bildern und den auf andere Weise gewonnenen Bildern (140) und Einrichtungen zum Bestimmen der Verschiebung der durch das Mikroskop beobachteten Punkte (1) relativ zu den Orten der Punkte in den auf andere Weise gewonnenen Bildern aufweist.

17. Vorrichtung nach Anspruch 16, **dadurch gekennzeichnet, dass** sie Einrichtungen (210) zum Bestimmen einer Verschiebungsfunktion durch Interpolation für die zwischen den markierten Punkten (1, 145) angeordneten Punkte aufweist.

18. Vorrichtung nach Anspruch 17, **dadurch gekennzeichnet, dass** die Einrichtungen (210) zum Bestimmen der Verschiebungsfunktion für Extrapolation über die markierten Punkte (1, 145) hinaus ausgebildet sind.

19. Vorrichtung nach Anspruch 17 oder 18, **dadurch gekennzeichnet, dass** sie Einrichtungen (210) zum Deformieren der auf andere weise gewonnenen Bilder (140) unter Verwendung der Verschiebungsfunktion auf solche Weise aufweist, dass die markierten Punkte (145) in den auf andere weise gewonnenen Bildern (140) mit den mit dem Mikroskop beobachteten entsprechenden Punkten (1) zusammenfallen.

## Claims

1. Method for observing objects from various viewing directions by means of a microscope having a device for electronically storing images and computer-controlled drive devices for a plurality of axes of motion, **characterized in that**
- a first recording of the target region of the object is created and stored,
- the microscope is pivoted by a defined angle,
- a second recording of the target region is created and stored,
- one and the same point of the object is marked in both recordings,
- the coordinates of the point are calculated by triangulation, and
- the control is set up in such a way that it is possible to carry out a pivot movement about the set point.

2. Method according to Claim 1, **characterized in that** the control is set up in such a way that translation movements of the microscope can be carried out.

3. Method according to Claim 1 or 2, **characterized in that** the acquisition and calculation are carried out for a plurality of points of the object.

4. Method according to one of Claims 1 to 3, **characterized in that** use is made of a microscope with devices for mirroring in data and images.

5. Method according to Claim 4, **characterized in that** the devices are designed for mirroring in data of the movement control.

6. Method according to Claim 4 or 5, **characterized in that** the devices are designed for mirroring in images of the object obtained in a different manner.

7. Method according to Claim 6, **characterized in that** the points which correspond to the image points marked in the stored microscope recordings are marked in the images of the object obtained in a different manner.

8. Method according to Claim 7, **characterized in that** shifts of the points observed by means of the microscope relative to the locations of the points in the images obtained in a different manner are determined.

9. Method according to Claim 8, **characterized in that** a shift function is determined by interpolation for the points arranged between the marked points.

10. Method according to Claim 9, **characterized in that** the shift function is extrapolated beyond the marked points.

11. Method according to Claim 9 or 10, **characterized in that** the images obtained in a different manner are deformed using the shift function in such a way that the marked points in the said images coincide with the corresponding points observed with the microscope.

12. Apparatus for carrying out the method according to one of Claims 1 to 11 with a microscope having a device for electronically storing images and computer-controlled drive devices for a plurality of axes of motion, **characterized in that** it has devices (31-36) for mirroring in control data (11, 12, 13) of the drive devices (204, 206, 208).

13. Apparatus according to Claim 12, **characterized in that** the drive devices (204, 206, 208) can be controlled in a wire-free manner by means of a sterilizable unit (115) having an actuation button (113) for the type of movement and an actuation button (113) for the execution of a movement step.

14. Apparatus according to Claim 12 or 13, **characterized in that** the computer control (210) is designed for effecting translational and pivot movements.

15. Apparatus according to one of Claims 12 to 14, **characterized in that** it has a device for mirroring images (140) of the object obtained (31-36) in a different manner into the image field (10) of the microscope.

16. Apparatus according to Claim 15, **characterized in that** it has devices (113) for marking points in the electronically stored images and the images (140) obtained in a different manner and devices for determining the shift of the points (1) observed by means of the microscope relative to the locations of the points in the images obtained in a different manner.

17. Apparatus according to Claim 16, **characterized in that** it has devices (210) for determining a shift function by interpolation for the points arranged between the marked points (1, 145).

18. Apparatus according to Claim 17, **characterized in that** the devices (210) are designed for determining the shift function for extrapolation beyond the marked points (1, 145).

19. Apparatus according to Claim 17 or 18, **characterized in that** it has devices (210) for deforming the images (140) obtained in a different manner using the shift function in such a way that the marked points (145) in the images (140) obtained in a different manner coincide with the corresponding points (1) observed with the microscope.

## Revendications

1. Procédé pour observer des objets à partir de différentes directions de visée avec un microscope, qui présente un dispositif pour la mémorisation électronique d'images et des dispositifs d'entraînement commandés par ordinateur pour plusieurs axes de déplacement, **caractérisé en ce que**
- une première prise de vue de la zone cible de l'objet est effectuée et mémorisée,
- le microscope est basculé d'un angle défini,
- une deuxième prise de vue de la zone cible est effectuée et mémorisée,
- un seul et même point de l'objet est marqué dans les deux prises de vue,
- les coordonnées du point sont calculés par triangulation, et
- la commande est aménagée de façon à pouvoir réaliser un mouvement de basculement autour de ce point.

2. Procédé selon la revendication 1, **caractérisé en ce que** la commande est aménagée de telle sorte que des déplacements de translation du microscope peuvent être effectués.

3. Procédé selon la revendication 1 ou 2, **caractérisé en ce que** l'enregistrement et le calcul sont effectués pour plusieurs points de l'objet.

4. Procédé selon l'une quelconque des revendications 1 à 3, **caractérisé en ce qu'**on utilise un microscope avec des dispositifs pour l'introduction par réflexion de données et d'images.

5. Procédé selon la revendication 4, **caractérisé en ce que** les dispositifs sont réalisés pour l'introduction par réflexion de données de la commande de déplacement.

6. Procédé selon la revendication 4 ou 5, **caractérisé en ce que** les dispositifs sont réalisés pour l'introduction par réflexion d'images de l'objet obtenues d'une autre façon.

7. Procédé selon la revendication 6, **caractérisé en ce que**, sur des images de l'objet obtenues d'une autre façon, on marque les points qui correspondent aux points d'image marqués dans les prises de vue faites au microscope et mémorisées.

8. Procédé selon la revendication 7, **caractérisé en ce que** des déplacements des points observés dans le microscope sont déterminés par rapport aux emplacements des points sur les images obtenues d'une autre façon.

9. Procédé selon la revendication 8, **caractérisé en ce qu'**une fonction de déplacement pour les points disposés entre les points marqués est déterminée par interpolation.

10. Procédé selon la revendication 9, **caractérisé en ce que** la fonction de déplacement est extrapolée au-delà des points marqués.

11. Procédé selon la revendication 9 ou 10, **caractérisé en ce que** les images obtenues d'une autre façon sont déformées avec l'utilisation de la fonction de déplacement de telle sorte que les points marqués dans les mêmes images coïncident avec les points correspondants observés au microscope.

12. Dispositif pour la mise en oeuvre du procédé selon l'une quelconque des revendications 1 à 11 avec un microscope, qui présente un dispositif pour la mémorisation électronique d'images et des dispositifs d'entraînement commandés par ordinateur pour plusieurs axes de déplacement, **caractérisé en ce qu'**il présente des dispositifs (31-36) pour l'introduction par réflexion de données de commande (11, 12, 13) des dispositifs d'entraînement (204, 206, 208).

13. Dispositif selon la revendication 12, **caractérisé en ce que** les dispositifs d'entraînement (204, 206, 208) peuvent être commandés sans fil avec une unité (115) stérilisable, qui présente un bouton d'actionnement (113) pour le type de déplacement et un bouton d'actionnement (113) pour l'exécution d'une étape de déplacement.

14. Dispositif selon la revendication 12 ou 13, **caractérisé en ce que** la commande par ordinateur (210) est réalisée pour l'exécution de mouvements de translation et de pivotement.

15. Dispositif selon l'une quelconque des revendications 12 à 14, **caractérisé en ce qu'**il présente un appareil pour l'introduction par réflexion d'images (140) de l'objet obtenues d'une autre façon (31-36) dans le champ d'image (10) du microscope.

16. Dispositif selon la revendication 15, **caractérisé en ce qu'**il présente des appareils (113) pour le marquage de points dans les images mémorisées de façon électronique et les images (140) obtenues d'une autre façon et des appareils pour la détermination du déplacement des points (1) observés dans le microscope par rapport aux emplacements des points dans les images obtenues d'une autre façon.

17. Dispositif selon la revendication 16, **caractérisé en ce qu'**il présente des appareils (210) pour la détermination d'une fonction de déplacement par interpolation pour les points disposés entre les points (1, 145) marqués.

18. Dispositif selon la revendication 17, **caractérisé en ce que** les appareils (210) pour la détermination de la fonction de déplacement pour l'extrapolation sont réalisés au-delà des points marqués (1, 145).

19. Dispositif selon la revendication 17 ou 18, **caractérisé en ce qu'**il présente des appareils (210) pour la déformation des images (140) obtenues d'une autre façon avec l'utilisation de la fonction de déplacement de telle façon que les points (145) marqués dans les images (140) obtenues d'une autre façon coïncident avec les points (1) correspondants et observées avec le microscope.
